# EUROPÄISCHE PATENTSCHRIFT

(11) **EP 1 066 029 B1**
(45) Veröffentlichungstag und Bekanntmachung des Hinweises auf die Patenterteilung: **09.10.2002**
(21) Anmeldenummer: 99913291.3
(22) Anmeldetag: 23.03.1999
(51) Int. Cl.: A61K 9/00, A61K 47/02

(54) **PERORALE WIRKSTOFF-SUSPENSION**
PERORAL ACTIVE AGENT SUSPENSION
PRINCIPE ACTIF EN SUSPENSION PERORALE

(30) Priorität: 27.03.1998 EP 98105568
(43) Veröffentlichungstag der Anmeldung: 10.01.2001
(73) Patentinhaber: Boehringer Ingelheim Pharma KG, 55218 Ingelheim am Rhein (DE)
(72) Erfinder: TÜRCK, Dietrich, D-89077 Ulm (DE); SCHMELMER, Veit, D-88400 Biberach (DE)
(74) Vertreter: Laudien, Dieter, Dr.
(86) Internationale Anmeldenummer: EP9901948
(87) Internationale Veröffentlichungsnummer: WO99049845

(56) Entgegenhaltungen:
- EP-A- 0 179 430
- EP-A- 0 560 329
- WO-A-98/17250
- US-A- 4 835 187

## Beschreibung

### 1. Einleitung

Die vorliegende Erfindung betrifft peroral applizierbare Suspensionen pharmazeutischer Wirkstoffe vom NSAID-Typ (nonsteroidal-antiinflammatory drugs), insbesondere jedoch des Antirheumatikums Meloxicam, sowie ein Verfahren zu deren Herstellung.

### 2. Problemstellung

Bei der per os-Applikation von Arzneistoffen finden unterschiedliche Arzneiformen Anwendung. So werden neben festen, einzeldosierten Formen wie beispielsweise Tabletten, Hartu. Weichgelatinekapseln auch flüssige Formen wie beispielsweise Lösungen und Säfte verabreicht, bei denen sich die zu verabreichende Dosis über das Applikationsvolumen einstellen läßt.

Lösungen und Säfte bieten dabei den Vorteil, daß sie auch von Patienten, die mit der Einnahme fester, einzeldosierter Formen Schwierigkeiten haben (z.B. Kinder, ältere Patienten), leicht und sicher eingenommen werden können. Für die Anwendung im Veterinärbereich bieten flüssige Darreichungsformen vorteilhaft einfache Dosierungsmöglichkeiten.

Hierbei ist jedoch zu beachten, daß selbst bei gleicher Dosierung und gleichem Applikationsweg die Wirkung ein und desselben Arzneistoffs unterschiedlich sein kann. Diese Unterschiede bewirken, daß der für einen Arzneistoff in einer bestimmten Zubereitung klinisch nachgewiesene therapeutische Effekt mit einer anderen Zubereitung desselben Arzneistoffs nicht zu erzielen ist, mithin innerhalb der Therapie nicht einfach die eine gegen die andere Zubereitung ausgetauscht werden kann. Solche therapeutisch nicht gleichwertigen Zubereitungen bezeichnet man als "nicht bioäquivalent". Für die p.o.-Applikation von Arzneiformen gilt, daß der Arzneistoff aus flüssigen Zubereitungen, insbesondere Lösungen, meist schneller resorbiert wird als aus Tabletten oder Kapseln und diese Arzneiformen dementsprechend nicht immer bioäquivalent sind (Bauer K. H.; Frömming K.-H.; Führer C., Pharmazeutische Technologie, 5. Auflage 1997, Gustav Fischer Verlag, Stuttgart, Seite 213).

Meloxicam ist ein zur Klasse der NSAID's zählendes Antirheumatikum. NSAID's sind Hemmstoffe der Cyclooxygenase, wobei für Meloxicam eine selektive Hemmung des lsoenzyms COX-2 und damit ein vermindertes Risiko unerwünschter gastro-intestinaler Nebenwirkungen nachgewiesen werden konnte. Für die sichere Applikation von Meloxicam und anderen Wirkstoffen, beispielsweise anderen NSAID's, ist eine flüssige p.o.-Darreichungsform als Alternative zur festen Form (Kapsel, Tablette) insbesondere in der Pädiatrie und im Veterinärbereich wünschenswert.

Die komplexe Aufgabe der vorliegenden Erfindung bestand primär in der Herstellung einer p.o. applizierbaren, flüssigen Zubereitung von Meloxicam. Die Formulierung sollte bei der Erstanwendung im akuten Fall einen schnellen Wirkungseintritt zeigen. Die Substanz wird jedoch bevorzugt zur Dauertherapie eingesetzt. Bei solchen Dauertherapien sollte die flüssige p.o. Formulierung im steady state bioäquivalent mit anderen p.o. Formulierungen (Tablette, Kapsel) sein, um die Therapie wahlweise mit einer flüssigen oder festen p.o.-Formulierung zu ermöglichen. Die flüssige Zubereitung sollte gleichzeitig einen guten Geschmack aufweisen, um von Kindern akzeptiert zu werden und auf diese Weise Einnahmetreue und Therapiesicherheit zu gewährleisten. Die flüssige Zubereitung sollte zusätzlich vorzugsweise keinen Ethanol enthalten, da ein schädlicher Einfluß von Ethanol auch in physiologisch unbedenklicher, nicht-toxischer Konzentration insbesondere bei Kindern nicht völlig auszuschließen ist. Überdies besteht bei der Verwendung von Ethanol die Gefahr des Mißbrauchs durch alkoholabhängige Patienten bzw. des Rückfalls bei ehemals alkoholabhängigen Patienten. Der Eignung der Formulierung für diabetische Patienten sollte Rechnung getragen-werden. Um eine exakte Dosierung einer flüssigen p.o.-Darreichungsform von Meloxicam zu gewährleisten, ist ferner die Homogenität der Zubereitung über einen ausreichend langen Zeitraum während der Entnahme aus dem Primärpackmittel zu gewährleisten.

Die Zubereitung sollte überdies auch bei Tieren anwendbar sein. Die Veterinärformulierung sollte ebenfalls eine für viele mit Antirheumatika behandelbare Tierarten, insbesondere Säugetierarten, geeigneten Geruch und Geschmack aufweisen, um aufgrund guter Akzeptanz Einnahmetreue und Therapiesicherheit zu gewährleisten.

### 3. Beschreibung der Erfindung

Eine naheliegende Möglichkeit zur Herstellung einer flüssigen p.o.-Formulierung eines pharmazeutischen Wirkstoffs stellt eine Lösung der Substanz in physiologisch indifferenten Lösungsmitteln (in erster Linie Wasser pharmazeutischer Qualität) dar. Dieser Ansatzpunkt ist in vielen Fällen jedoch ungeeignet. Um den angestrebten attraktiven Geschmack einer flüssigen p.o.-Formulierung beispielsweise von Meloxicam zu gewährleisten, lassen sich Lösungen von Meloxicam nicht verwenden, da die Substanz in gelöstem Zustand einen unangenehmen Eigengeschmack aufweist. Dieser Eigengeschmack tritt in allen für die p.o.-Applikation von Lösungen einsetzbaren Lösungsmitteln auf und läßt sich auch durch Zusatz von Geschmackskorrigentien wie Aromen und Süßstoffen nur unzureichend maskieren.

Dagegen zeigt Meloxicam keinen wahrnehmbaren Eigengeschmack, wenn die Substanz für eine flüssige p.o.-Zubereitung in einem physiologisch indifferenten Dispersionsmedium suspendiert wird und die Löslichkeit von Meloxicam im verwendeten Dispersionsmedium sehr gering ist. Dadurch ergab sich ein geeigneter Ansatzpunkt zur Lösung der gestellten Aufgabe. Dieser Ansatzpunkt läßt sich analog auf andere Wirkstoffe der NSAID-Klasse übertragen. Da bei einer Konzentration von über 500 µg/ml gelöstem Meloxicam bereits ein deutlich wahrnehmbarer, unangenehmer Geschmack wahrnehmbar ist. muß die Löslichkeit dieses Wirkstoffs in den verwendbaren Dispersionsmedien unterhalb dieses Grenzwertes liegen.

Bei der Verwendung einer Wirkstoff-Suspension ergibt sich das Problem, daß die Homogenität der Suspension über einen ausreichend langen Zeitraum während der Entnahme aus dem Primärpackmittel (z.B. Glasflasche, 100 ml) gewährleistet sein muß, um eine exakte Dosierung sicherzustellen. Die Sedimentation von in flüssigen Medien dispergierten Feststoffen läßt sich jedoch nicht verhindern, sondern nur über einen mehr oder weniger langen Zeitraum verzögern. Ein konventioneller Ansatzpunkt zur Verzögerung der Sedimentation ist beispielsweise die Viskositätserhöhung des Dispergiermediums durch geeignete Zusätze, beispielsweise der Zusatz von organischen Hydrokolloidbildnern, beispielsweise Celluloseether, oder von Siliciumdioxid als Verdickungsmittel. Die Erhöhung der Viskosität des Dispergiermittels hat jedoch den entscheidenden Nachteil, daß die Redispergierbarkeit des entstehenden Sediments wesentlich verschlechtert wird, sodaß bei einer zu hohen Viskosität der Suspension eine Rekonstitution der Suspension unmöglich wird. Ferner ist eine bei Lagerung der Suspension durch Kontakt der Einzelteilchen unter Schwerkraft zu beobachtende Kuchenbildung (Caking) zu vermeiden. Literaturbekannt für die Vermeidung des Cakings ist beispielsweise die gezielte Flockung solcher Systeme mittels Adsorption von potentialbestimmenden lonen (Sucker H., Fuchs P., Speiser P., Pharmazeutische Technologie, 5. Auflage 1991, Georg Thieme Verlag, Stuttgart, S. 423). Der industriellen Herstellung stabiler Suspensionen durch gezielte Flockung sind Grenzen gesetzt. da optimale Eigenschaften solcher Suspensionssysteme aufgrund der Variabilität des suspendierten Feststoffs schwer reproduzierbar sind und die Suspensionsstabilität wesentlich von den verwendeten Hilfsstoffen beeinflußt wird.

Im US-Patent 4,835,187 wird die Herstellung einer sprühgetrockneten Ibuprofen-Pulverformulierung, enthaltend neben dem Wirkstoff kolloidales Siliziumdioxid und einen wasserlöslichen Celluloseether, sowie eine daraus hergestellte schnell freisetzende feste Darreichungsform beschrieben. In einem Zwischenschritt des Herstellverfahrens wird dabei eine wäßrige Suspension, enthaltend u. a. die oben genannten Bestandteile, erhalten.

Überraschenderweise läßt sich die Suspension eines pharmazeutischen Wirkstoffs des NSAID-Typs durch Zusatz von geringen Mengen hochdispersem Siliciumdioxid bei Gegenwart geringer Mengen von hydrophilen Polymeren stabilisieren. Aufgrund der geringen Konzentration von hochdispersem Siliciumdioxid und hydrophilen Polymeren im Dispersionsmedium ist die Viskosität gering; eine unerwünschte, die Rekonstitution der Suspension behindernde Erhöhung der Viskosität durch gelartige Verdickung des Dispersionsmediums tritt nicht ein, sofern dem Dispersionsmedium gleichzeitig geringe Mengen von im Dispersionsmedium löslichen hydrophilen Polymeren zugesetzt werden und das Siliciumdioxid mit Hilfe hoher Scherkräfte in die Suspension eingetragen wird. Ausreichend hohe Scherkräfte können mit einem geeigneten scherintensiven Homogenisiermischer erzeugt werden, beispielsweise mit den Mischern der Modellreihe "Becomix", Firma A. Berents GmbH & Co. KG, Henleinstr. 19, D-28816 Stuhr, welche über schnelldrehende Homogenisatoren vom Rotor-Stator-Prinzip verfügen. Hierbei ist eine Rotor-Umfangsgeschwindigkeit von etwa 25 bis 27 m/s zur Erzeugung ausreichend hoher Scherkräfte besonders geeignet und wird zum Eintrag des hochdispersen Siliciumdioxids in das Dispersionsmittel während ca. 10 - 15 min zum Beispiel mit den Mischern Becomix RW 15 / RW 60 / RW 1000 verwendet. Hierdurch wird eine-besondere Siloid-Struktur aufgebaut. die in einem schwammartigen, mit Hohlräumen durchzogenen, dreidimensionalen Gerüst von hydratisiertem hochdispersen Siliciumdioxid besteht, an die der Wirkstoff adsorbiert werden kann.

Geeignetes hochdisperses Siliciumdioxid hat beispielsweise eine spezifische Oberfläche von mindestens 50 m²/g, vorzugsweise 100 bis 400 m²/g, besonders bevorzugt ist eine spezifische Oberfläche von etwa 200 m²/g (z.B. Aerosil® 200).

Gegenstand der Erfindung ist eine pharmazeutische Zusammensetzung in Form einer oral applizierbaren Suspension umfassend einen Wirkstoff vom NSAID-Typ aus der Gruppe der Propionsäurederivate, der Essigsäurederivate, der Fenaminsäurederivate, der Biphenylcarbonsäurederivate, der sauren Enolcarboxamide, der Diaryl-Heterocyclen mit Methylsulfonyl- oder Aminosulfonyl-Substituenten und der sauren Sulfonamide und deren pharmazeutisch verträglichen Salze, mit einem suspensionsfähigen Korngrößenspektrum, in dem mindestens 90 % der Partikel kleiner als 50 µm sind, in einem physiologisch indifferenten Dispersionsmedium, in dem die Löslichkeit des Wirkstoffs kleiner als 500 µg/ml ist, sodaß die Suspension keinen wahrnehmbaren Eigengeschmack aufweist, dadurch gekennzeichnet, daß die Suspension 0,1 bis 5 Gew.-% hochdisperses Siliciumdioxid mit einer spezifischen Oberfläche von mindestens 50 m²/g zur Stabilisierung durch Ausbildung einer dreidimensionalen Siloidstruktur enthält, wobei die dreidimensionale Siloidstruktur mittels Eintragung des Siliciumdioxids in das Dispersionsmedium unter Einwirkung von Scherkräften, charakterisiert durch eine Rotor-Umfangsgeschwindigkeit von 15 bis 35 m/s, erzeugt wird und die Suspension 0,05 bis 2 Gew.-% eines wasserlöslichen Celluloseethers in pharmazeutischer Qualität enthält.

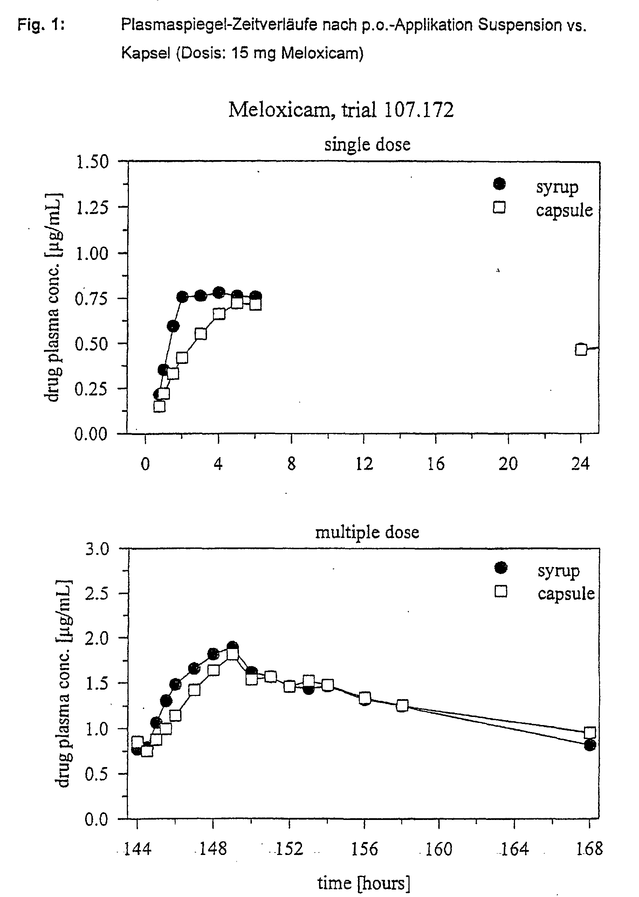

Die vorstehend erwähnte dreidimensionale Siloid-Struktur besteht aus vernetzten, gequollenen und kohärenten Strängen von SiO₂, zwischen denen größere, mit Dispersionsmedium gefüllte Hohlräume erkennbar sind. Die suspendierten Feststoffpartikel des Wirkstoffs, beispielsweise des Meloxicams, sind dabei fast ausschließlich an die SiO₂-Stränge adsorbiert. Auf diese Weise werden die suspendierten Partikel schnell und vollständig benetzt, eine Agglomeration der Arzneistoffpartikel kann vollständig verhindert werden. Es resultiert eine Suspension des Wirkstoffs mit hervorragender Homogenität und Dosiergenauigkeit. Die beschriebene Siloidstruktur bewirkt keine gelartige Verdickung des Dispersionsmediums, sondern führt zu einer niedrigviskosen, gießfähigen Suspension. Zur Veranschaulichung der beschriebenen Siloid-Struktur dienen die Abbildungen 1 bis 3.

Gleichzeitig wirkt die dreidimensionale Siloid-Struktur als Sedimentationsstabilisator. Die beschriebene Struktur ist sehr voluminös und wird auch durch Sedimentation nur unwesentlich und sehr langsam verdichtet. So verringert sich das Volumen der Siloid-Struktur auch nach monatelanger Lagerung nur um ca. 20%. Die Volumenreduktion durch Sedimentation führt nicht zu einem unerwünschten Caking, das Sediment ist durch extrem geringe mechanische Kräfte (z.B. ein sehr leichtes Schütteln einer in handelsübliche Glasflaschen abgefüllten p.o.-Suspension von Meloxicam) rasch und leicht redispergierbar. Die verlangsamte Sedimentation garantiert dem Anwender eine ausreichende Zeitspanne für die Entnahme von homogenen Einzeldosen der erfindungsgemäßen p.o.-Suspension eines pharmazeutischen Wirkstoffs aus dem Primärpackmittel, wodurch die Dosiergenauigkeit gesichert ist.

Beispielsweise enthält eine erfindungsgemäße Wirkstoff-Suspension 0,1 - 5 Gew.-% hochdisperses Siliciumdioxid (z.B. Aerosil® 200), vorzugsweise 0,5 - 2 Gew.-%, insbesondere jedoch 0,5 - 1,5 Gew.-%.

Als lösliche hydrophile Polymeren sind Celluloseether in pharmazeutischer Qualität geeignet, beispielsweise Hydroxyethylcellulose (HEC), Hydroxyproylcellulose (HPC) und Hydroxypropylmethylcellulose (HPMC). Bevorzugt ist Hydroxyethylcellulose. Beispielsweise enthält eine erfindungsgemäße Wirkstoff-Suspension 0,05 - 2 Gew.-% wasserlösliche Celluloseether, vorzugsweise 0,05 - 0,5 Gew.-%, insbesondere jedoch 0,05 - 0,1 Gew.-%.

Ganz besonders bevorzugt enthält die erfindungsgemäße Wirkstoff-Suspension 0.5 - 1,5 Gew.-% hochdisperses Siliciumdioxid und 0,05 - 0,1 Gew.-% Hydroxyethylcellulose.

Die Eigenschaften einer erfindungsgemäßen flüssigen p.o.-Wirkstoff-Suspension werden stark von der Teilchengröße des suspendierten Wirkstoffs beeinflußt. Um den bei Einmalgabe gewünschten schnellen Wirkungseintritt zu erzielen, ist eine geringe Teilchengröße erforderlich, die eine möglichst rasche Auflösung des Wirkstoffs im Gastrointestinal-Trakt gewährleistet. Im Korngrößenspektrum des für eine erfindungsgemäße Suspension geeigneten Wirkstoffs sind daher mindestens 90% der Partikel kleiner als 50 µm, vorzugsweise sind mindestens 50% der Partikel kleiner als 10 µm, besonders bevorzugt sind etwa 90% der Partikel kleiner als 10 µm (bestimmt z.B. mittels Laserdiffraktometrie). Eine entsprechend feindisperse Arzneistoffqualität läßt sich auf einfache Weise durch geeignetes Mahlen einer grobdispersen Qualität erzielen. Geeignete Mühlen für ein entsprechendes Mahlverfahren sind beispielsweise handelsübliche Strahlmühlen.

Die beschriebene geringe Teilchengröße des Wirkstoffs in einer erfindungsgemäßen Suspension bietet überdies den Vorteil einer geringen Sedimentationsgeschwindigkeit der suspendierten Partikel, was sich günstig auf die Homogenität der beschriebenen flüssigen P.O.-Formulierung des Wirkstoffs auswirkt und dementsprechend eine hohe Dosiergenauigkeit gewährleistet.

Die Löslichkeit des Wirkstoffs in geeigneten physiologisch verträglichen Dispersionmedien sollte weniger als 500 µg/ml betragen. Vorzugsweise beträgt die Löslichkeit maximal 50 µg/ml, besonders bevorzugt ist eine Löslichkeit von maximal 5 µg/ml, insbesondere jedoch maximal 0,5 µg/ml.

Es ist dem Fachmann ohne weiteres möglich, für einen gegebenen Wirkstoff des NSAID-Typs ein geeignetes physiologisch verträgliches Dispersionsmedium zu ermitteln, in dem der Wirkstoff die vorstehend angegebenen Löslichkeits-Eigenschaften aufweist. Für Meloxicam besteht das physiologisch verträgliche Dispersionmedium vorzugsweise in einem wäßrigen Puffersystem im pH-Bereich 2-4.

Eine erfindungsgemäße p.o. applizierbare Suspension kann als pharmazeutischen Wirkstoff ein oder mehrere NSAID's enthalten. Als NSAID's seien die Wirkstoffe der folgenden Klassen erwähnt:
(1) Propionsäurederivate,
(2) Essigsäurederivate,
(3) Fenaminsäurederivate,
(4) Biphenylcarbonsäurederivate,
(5) saure Enolcarboxamide,
(6) Diaryl-Heterocyclen mit Methylsulfonyl- oder Aminosulfonyl-Substituenten und
(7) saure Sulfonamide.

Als Propionsäurederivate seien beispielsweise folgende Wirkstoffe genannt, wobei die Aufzählung keine Limitation dieser Wirkstoffklasse bedeuten soll:

Ibuprofen, Naproxen, Flurbiprofen, Fenoprofen, Fenbufen, Ketoprofen, Indoprofen, Pirprofen, Carprofen, Oxaprozin, Pranoprofen, Miroprofen, Tioxaprofen, Suprofen, Alminoprofen, Tiaprofensäure und Fluprofen oder deren pharmazeutisch verträglichen Salze.

Als Essigsäurederivate seien beispielsweise folgende Wirkstoffe genannt, wobei die Aufzählung keine Limitation dieser Wirkstoffklasse bedeuten soll:

Indomethacin, Sulindac, Tolmetin, Zomepirac, Nabumetone, Diclofenac, Fenclofenac, Alclofenac, Bromfenac, Ibufenac, Aceclofenac, Acemetacin, Fentiazac, Clidanac, Etodolac und Oxpinac oder deren pharmazeutisch verträglichen Salze.

Als Fenaminsäurederivate seien beispielsweise folgende Wirkstoffe genannt, wobei die Aufzählung keine Limitation dieser Wirkstoffklasse bedeuten soll:

Mefenaminsäure, Meclofenaminsäure, Flufenaminsäure, Nifluminsäure und Tolfenaminsäure oder deren pharmazeutisch verträglichen Salze.

Als Biphenylcarbonsäurederivate seien beispielsweise folgende Wirkstoffe genannt, wobei die Aufzählung keine Limitation dieser Wirkstoffklasse bedeuten soll:

Diflunisal und Flufenisal oder deren pharmazeutisch verträglichen Salze.

Als saure Enolcarboxamide (Oxicame) seien beispielsweise folgende Wirkstoffe genannt, wobei die Aufzählung keine Limitation dieser Wirkstoffklasse bedeuten soll:

Piroxicam, Tenoxicam, Lornoxicam und Meloxicam oder deren pharmazeutisch verträglichen Salze.

Als saures Sulfonamid sei beispielsweise Nimesulide genannt, wobei dies keine Limitation dieser Wirkstoffklasse bedeuten soll.

Chemische Strukturen, pharmakologische Aktivität, Nebeneffekte und Angaben über übliche Dosierungsbereiche zu vorstehend genannten NSAID's sind beispielsweise angegeben in Physician's Desk Reference, 35th Edition, 1981; The Merck Index, 12th Edition, Merck and Company, Rahway, New Jersey (1996); Cutting's Handbook of Pharmacology, 6th Edition, Ed. T. Z. Csacky, M.D., Appleton-Century-Crofts, New York, 1979, Chapter 49:538-550.

Eine Dosierungseinheit kann für die folgenden NSAID's beispielsweise betragen:
100 - 500 mg Diflunisal, 25 - 100 mg Zomepirac-Natrium, 50 - 400 mg Ibuprofen, 125 - 500 mg Naproxen, 25 - 100 mg Flurbiprofen, 50 - 100 mg Fenoprofen, 10 - 20 mg Piroxicam,
5 - 20 mg Meloxicam, 125 - 250 mg Mefenaminsäure, 100 - 400 mg Fenbufen und 25 - 50 mg Ketoprofen.

Besonders bevorzugte erfindungsgemäße p.o. applizierbare Suspensionen sind solche, die als Wirkstoff ein saures Enolcarboxamid, insbesondere Meloxicam, enthalten.

Meloxicam ist ein NSAID vom Strukturtyp einer Enolsäure und zeigt eine deutlich pH-abhängige Löslichkeit. Die minimale Löslichkeit liegt in gepufferten wäßrigen Systemen bei pH-Werten von 2-4. Die Löslichkeit beträgt dabei in diesem pH-Bereich weniger als 0,5 µg/ml (Luger P., Daneck K., Engel W., Trummlitz G., Wagner K., Structure and physicochemical properties of meloxicam, a new NSAID, Eur. J. Pharm. Sci. 4 (1996), 175-187).

Als Dispersionsmedium für eine erfindungsgemäße flüssige p.o.-Suspension von Meloxicam eignen sich demnach physiologisch verträgliche wäßrige Puffersysteme im pH-Bereich von 2-4, deren Mischungen untereinander oder deren Mischungen mit weiteren physiologisch verträglichen Flüssigkeiten, die zusätzlich zur Verbesserung von Detail-Eigenschaften der Meloxicam-Suspension geeignet sind, insbesondere
- zur Einstellung der Viskosität des Dispersionsmediums, um damit die Sedimentationsgeschwindigkeit der suspendierten Arzneistoffpartikel zu verringern,
- zur Gewährleistung eines attraktiven Geschmacks der flüssigen p.o.-Formulierung und
- zur Erleichterung der Benetzbarkeit der suspendierten Arzneistoffpartikel.

Als weitere physiologisch verträgliche Flüssigkeiten im Sinne der beschriebenen Erfindung eignen sich vorzugsweise Glycerol und gegebenenfalls wäßrige Lösungen von Zuckeralkoholen wie Sorbitol, Mannitol und Xylitol, sowie deren Mischungen. Diese Stoffe haben in einer erfindungsgemäßen Suspension den Vorteil
- einer Erhöhung der Viskosität des Dispersionsmediums und damit einer Verringerung der Sedimentationsgeschwindigkeit der suspendierten Arzneistoffpartikel sowie einer erleichterten Handhabung beim Ausbringen der flüssigen Formulierung in Dosierhilfen (z.B. handelsübliche Dosierlöffel oder spezielle Dosiersysteme, wie z.B. Dosierspritzen) und beim Ausbringen der flüssigen Formulierung durch Tropfdosierung (z.B. handelsübliche Tropfeinsätze),
- ihres leicht süßen Eigenschmacks, der einen attraktiven Geschmack der flüssigen p.o.-Formulierung gewährleistet,
- ihrer Eignung für diabetische Patienten und für mit Anitirheumatika behandelbare Tiere sowie
- einer Erleichterung der Benetzbarkeit der suspendierten Arzneistoffpartikel.

Geeignete physiologisch verträgliche wäßrige Puffersysteme im pH-Bereich 2-4 sind beispielsweise Natriumdihydrogen-phosphat Dihydrat / Citronensäure Monohydrat-Puffer, Glycin/HCl (S. P. Sørensen, Biochem. Z., 21, 131 (1909); Biochem. Z., **22,** 352 (1909)), Na-Citrat/HCl (S. P. Sørensen, Biochem. Z., **21**, 131 (1909); Biochem. Z., **22,** 352 (1909)), K-Hydrogenphthalat/HCl (Clark und Lubs, J. Bact., **2**, 1 (1917)), Citronensäure/Phosphat (T. C. Mcllvaine, J. Biol. Chem., **49**, 183 (1921)), Citrat-Phosphat-Borat/HCl (Teorell und Stenhagen, Biochem. Z., **299,** 416 (1938)) und Britton-Robinson Puffer (Britton und Welford, J. Chem. Soc., **1**, 1848 (1937)).

Bevorzugt basiert ein Dispersionsmedium für eine erfindungsgemäße flüssige p.o.-Suspension von Meloxicam auf einem wäßrigen Puffersystem im pH-Bereich von 2-4 in Mischung mit einer oder mehreren der physiologisch verträglichen Flüssigkeiten Glycerol und wäßrigen Lösungen der Zuckeralkohole Mannitol, Sorbitol und Xylitol.

Beispielsweise besteht das Dispersionsmedium einer erfindungsgemäßen flüssigen p.o.-Suspension von Meloxicam aus Mischungen von ca. 30-50% wäßrigem Puffersystem im pH-Bereich 2-4, wobei wäßriger Natriumdihydrogenphosphat Dihydrat / Citronensäure Monohydrat-Puffer bevorzugt ist, ca. 10-20% Glycerol, ca. 10-20% Xylitol und ca. 20-30% Sorbitol- oder Mannitol-Lösung (70% Sorbitol oder Mannitol in Wasser). Glycerol und die genannten Zuckeralkohole können jeweils einzeln oder auch im Gemisch untereinander im Dispersionsmedium enthalten sein.

Die gebrauchsfertige Suspension kann unterschiedliche Mengen des Wirkstoffs Meloxicam enthalten, beispielsweise 0,050 bis 3,000 g/118 g, vorzugsweise 0,050 bis 2,000 g/118 g, insbesondere jedoch 0,050 bis 1,5 g/118 g, bezogen auf die Masse der applikationsfertigen Darreichungsform.

Zur weiteren Verbesserung des Geschmacks können einer erfindungsgemäßen flüssigen p.o.-Suspension eines oder mehrere Aromen und/oder ein oder mehrere Süßstoffe zugesetzt sein.

Als Aromen kommen beispielsweise flüssige und pulverförmige, wasserlösliche natürliche und naturidentische Aromen in Frage. Besonders bevorzugt sind flüssige Aromen, insbesondere die Aromen Himbeere, Erdbeere und Honig.

Als Süßstoffe kommen beispielsweise Saccharin-Natrium, Saccharin, Cyclamat, Acesulfam-Kalium und Taumatin in Fage.

Ferner können einer erfindungsgemäßen flüssigen p.o.-Suspension übliche Hilfsstoffe und/oder im pH-Bereich wirksame Konservierungsmittel, im Falle des Wirkstoffs Meloxicam vorzugsweise Natriumbenzoat, zugesetzt sein.

Durch Ausbildung der dreidimensionalen Siloidstruktur und der Adhäsion der suspendierten Feststoff-Partikel kann auf den Zusatz von Tensiden zur Verbesserung der Benetzbarkeit gänzlich verzichtet werden. Tenside können sich in Suspensionen negativ auswirken, da die Löslichkeit des Feststoffs im Dispersionsmedium teilweise erhöht sein kann, was zu einem unerwünschten Teilchengrößenwachstum führen kann. Auch haben Tenside, insbesondere ionische Tenside, häufig allergisierendes und/oder schleimhautreizendes Potential.

Ein weiterer Gegenstand der Erfindung ist ein Verfahren zur Herstellung einer p.o. applizierbaren, flüssigen Zubereitung eines pharmazeutischen Wirkstoffs des NSAID-Typs in Form einer stabilisierten Suspension, dadurch gekennzeichnet, daß
(i) der feste Wirkstoff gemahlen wird, sodaß ein Korngrößenspektrum erzeugt wird, in dem mindestens 90% der Partikel kleiner als 50 µm sind, vorzugsweise jedoch mindestens 50% der Partikel kleiner als 10 µm sind, insbesondere jedoch etwa 90% der Partikel kleiner als 10 µm sind,
(ii) der gemahlene Wirkstoff in einem physiologisch indifferenten Dispersionsmedium, in dem die Löslichkeit des Wirkstoffs geringer als 500 µg/ml ist, suspendiert wird,
(iii) in das Dispersionsmedium unter Anwendung hoher Scherkräfte, charakterisiert durch eine Rotor-Umfangsgeschwindigkeit von 15 bis 35 m/s, 0,1 bis 5,0 Gew.-% hochdispersen Siliciumdioxids, bezogen auf das Gewicht der applikationsfertigen Suspension, eingetragen werden,
(iv) dem Dispersionsmedium 0,05 bis 2 Gew.-% eines wasserlöslichen Celluloseethers, bezogen auf das Gewicht der applikationsfertigen Suspension, zugesetzt werden und
(v) dem Dispersionsmedium optional unabhängig voneinander eines oder mehrere Aromen, ein oder mehrere Süßstoffe, übliche Hilfsstoffe oder ein oder mehrere Konservierungsmittel zugesetzt werden können, wobei der Zusatz der Aromen aufgrund ihrer schaumbrechenden Eigenschaften vorzugsweise im letzten Schritt der Herstellung erfolgt.

Eine bevorzugte Ausführungsform des erfindungsgemäßen Verfahrens ist dadurch gekennzeichnet, daß der Wirkstoff Meloxicam ist und
(i) ein Komgrößenspektrum erzeugt wird, in dem etwa 90% der Partikel kleiner als 10 µm sind,
(ii) der gemahlene Wirkstoff in einem physiologisch verträglichen, wäßrigen Puffersystem im pH-Bereich von 2-4, beispielsweise Natriumdihydrogen-phosphat Dihydrat / Citronensäure Monohydrat-Puffer, Glycin/HCl, K-Hydrogenphthalat/HCl, Citronensäure/Phosphat, Citrat-Phosphat-Borat/HCl oder Britton-Robinson Puffer, deren Mischungen untereinander oder deren Mischungen mit weiteren physiologisch verträgliche Flüssigkeiten wie Glycerol oder gegebenenfalls wäßrigen Lösungen von Zuckeralkohole wie Sorbitol, Mannitol und Xylitol, suspendiert wird,
(iii) mit Hilfe eines Mischers unter Anwendung hoher Scherkräfte, charakterisiert durch eine Rotor-Umfangsgeschwindigkeit von 15 bis 35 m/s, vorzugsweise von 20 bis 30 m/s, in das Dispersionsmedium 0,1 bis 5,0 Gew.-% hochdisperses Siliciumdioxid, bezogen auf das Gewicht der applikationsfertigen Suspension, eingetragen werden,
(iv) dem Dispersionsmedium als wasserlöslicher Celluloseether Hydroxyethylcellulose in pharmazeutischer Qualität in einer Menge von 0,05 bis 2 Gew.-%, bezogen auf das Gewicht der applikationsfertigen Suspension, zugesetzt wird und
(v) dem Dispersionsmedium optional unabhängig voneinander eines oder mehrere Aromen, ein oder mehrere Süßstoffe, übliche Hilfsstoffe oder ein oder mehrere Konservierungsmittel zugesetzt werden können.

Eine besonders bevorzugte Ausführungsform des erfindungsgemäßen Verfahrens ist dadurch gekennzeichnet, daß
(i) ein Korngrößenspektrum erzeugt wird, in dem etwa 90% der Partikel kleiner als 10 um sind,
(ii) der gemahlene Wirkstoff in einem Dispersionsmedium bestehend aus Mischungen von 30-50% wäßrigem Puffersystem mit einem pH im Bereich von 2-4, wobei wäßriger Natriumdihydrogen-phosphat Dihydrat / Citronensaure Monohydrat-Puffer bevorzugt ist, 10-20% Glycerol, 10-20% Xylitol und 20-30% Sorbitol- oder Mannitol-Lösung (70% Sorbitol oder Mannitol in Wasser) suspendiert wird, wobei Glycerol und die genannten Zuckeralkohole jeweils einzeln oder auch im Gemisch untereinander im Dispersionsmedium enthalten sein können,
(iii) mit Hilfe eines Mischers unter Anwendung hoher Scherkräfte, beispielsweise charakterisiert durch eine Rotor-Umfangsgeschwindigkeit von 20 bis 30 m/s, vorzugsweise von etwa 25 bis 27 m/s, in das Dispersionsmedium 0,5-2,0 Gew.-% hochdisperses Siliciumdioxid, bezogen auf das Gewicht der applikationsfertigen Suspension, eingetragen werden,
(iv) dem Dispersionsmedium als hydrophile Polymere wasserlösliche Celluloseether in pharmazeutischer Qualität, vorzugsweise Hydroxyethylcellulose, in einer Menge von 0,05 - 0,5 Gew.-%, bezogen auf das Gewicht der applikationsfertigen Suspension, zugesetzt werden und
(v) dem Dispersionsmedium optional unabhängig voneinander eines oder mehrere Aromen, ein oder mehrere Süßstoffe, übliche Hilfsstoffe oder ein oder mehrere Konservierungsmittel zugesetzt werden können.

Bei allen genannten Ausführungsformen des Verfahrens erfolgen die Schritte (ii) bis (v) vorzugsweise unter Vakuum, da durch Lufteintrag infolge von Aufschwemmeffekten oder durch Luftabsorption an der Siloid-Struktur die Dichte der herzustellenden Suspension verändert und Inhomogenitäten erzeugt werden können.

Eine ganz besonders bevorzugte Ausführungsform des erfindungsgemäßen Verfahrens ist dadurch gekennzeichnet, daß der Wirkstoff ein saures Enolcarboxamid ist, insbesondere Meloxicam.

### 5. Beispiel

Die folgende Rezeptur bezieht sich auf die Herstellung von 100 ml einer erfindungsgemäßen, flüssigen, p.o.-applizierbaren Suspension von Meloxicam. Die Formulierung ermöglicht die mehrfache Entnahme einer Dosis von 7,5 mg Meloxicam in einem Volumen von 5 ml aus einer entsprechend dimensionierten Glasflasche durch Ausgießen in handelsübliche Dosierlöffel aus Kunststoff. Die für die Wirksamkeit und die Ausbildung der erfindungsgemäßen Siloidstruktur relevanten Inhaltsstoffe sind quantitativ angegeben, alle anderen Inhaltsstoffe können entsprechend den vorstehenden Angaben in der Formulierung enthalten sein.

Zum Schutz vor mikrobieller Kontamination während der Anwendung (Mehrdosenbehältnis) muß die Zubereitung ausreichend mit einem auf den pH-Bereich des Dispersionsmediums abgestimmten Konservierungsmittel (hier: Natriumbenzoat) konserviert sein.

| **Bestandteil** | | **Formulierung A** **g/100 ml (= g/118 g)** | **Formulierung B** **g/100 ml (= g/118 g)** |
|---|---|---|---|
| (1) | Meloxicam, strahlgemahlen | 0,150 | 1,500 |
| (2) | Siliciumdioxid, hochdispers | 1,000 | 1,500 |
| (3) | Hydroxyethylcellulose | 0,100 | 0,050 |

Weitere Inhaltsstoffe sind:

Sorbitol-Lösung 70% (nicht-krist.), Glycerol 85 %, Xylitol, Natriumdihydrogen-phosphat Dihydrat, Citronensäure Monohydrat, Saccharin Natrium krist., Natriumbenzoat und Himbeeraroma D 9599 (Formulierung A) oder Honigaroma 203108 (Formulierung B). Es wird mit gereinigtem Wasser auf ein Endvolumen von 100 ml entsprechend 118,000 g aufgefüllt.

### 5.1 Physikalisch-chemische Eigenschaften der Zubereitung

| | Formulierung A | Formulierung B |
|---|---|---|
| pH | 3,5 - 4,5 | 3,5 - 4,5 |
| Dichte | 1,16 -1,20 g/ml (20 °C) | 1,16 - 1,20 g/ml (20 °C) |
| Viskosität | 40 - 150 mPas | 60 - 200 mPas |

### 5.2 Pharmakokinetische Eigenschaften der Zubereitung

Ein Hauptziel für die Entwicklung einer flüssigen, p.o.-Formulierung von Meloxicam war der rasche Wirkungseintritt bei der Erstanwendung. Voraussetzung hierfür ist ein möglichst rasches Anfluten des Arzneistoffs in das zentrale Blutkompartiment.

Bei der Formulierung nach vorstehendem Beispiel ist dies erreicht. Im direkten Vergleich zwischen einer erfindungsgemäßen Suspension und einer Kapsel gleicher Dosis liegt der Zeitpunkt für die maximale Plasmakonzentration bei Einmalgabe von Meloxicam bei tₘₐₓ = 2 h (1,5 - 5 h; Suspension ) gegenüber tₘₐₓ = 5 h (2 - 6 h; Kapsel).

Im steady state sollte aufgrund der erwünschten therapeutischen Gleichwertigkeit der erfindungsgemäßen Suspension und einer festen p.o.-Formulierung Bioäquivalenz nachweisbar sein. Auch dies konnte im direkten Vergleich einer erfindungsgemäßen Suspension und einer Kapsel gleicher Dosis gezeigt werden. Im steady state liegen die maximalen Plasmaspiegel bei t_{max (ss)} = 5,0 h (5 - 9 h; Suspension ) bzw. t_{max (ss)} = 5,0 h (3 - 7 h; Kapsel). Eine graphische Darstellung der Studienergebnisse findet sich in Figur 1.

### 6. Herstellverfahren

Erfindungsgemäße Suspensionen lassen sich in einem mehrstufigen Misch- und Homogenisierverfahren herstellen. Entscheidend für die Ausbildung einer homogenen Suspension mit der beschriebenen Siloid-Struktur ist die Verwendung scherintensiver Homogenisiermischer, die eine Verteilung der zu suspendierenden Feststoffpartikel des Wirkstoffs und des hochdispersen Siliciumdioxids im Dispersionsmedium innerhalb einer sehr kurzen Zeit erlauben. Als besonders geeignet in diesem Sinne haben sich unterschiedlich dimensionierte Prozeßmischanlagen der Modellreihe "Becomix" (Firma A. Berents GmbH & Co. KG, Henleinstr. 19, D-28816 Stuhr) erwiesen, mit denen sich erfindungsgemäße Suspensionen in Ansatzgrößen von 2,5 bis 1.000 kg herstellen lassen. Diese Mischer verfügen über schnelldrehende Homogenisatoren vom Rotor-Stator-Prinzip, die eine optimale Durchmischung und Ausbildung der beschriebenen dreidimensionalen Siloid-Struktur gewährleisten. Ausreichend hohe Scherkräfte werden beispielsweise bei einer Rotor-Umfangsgeschwindigkeit von 20 bis 30 m/s, vorzugsweise von etwa 25 bis 27 m/s, erzeugt. Mit den Mischern Becomix RW 60 / RW 1000 werden zum Eintrag des hochdispersen Siliciumdioxids in das Dispersionsmittel Rotor-Umfangsgeschwindigkeiten von etwa 26 m/s während ca. 10 - 15 min verwendet.

### 1. Schritt (Vormischung)

Der Wirkstoff und das hochdisperse Siliciumdioxid werden in einem geeigneten Behälter (z.B. VA-Behälter) homogen vorgemischt. Diese Vormischung ist erforderlich, um eine bessere Benetzbarkeit des Arzneistoffs und eine agglomeratfreie Verteilung in der Suspension zu erreichen.

### 2. Schritt (Polymerlösung)

In einem geeigneten Ansatzbehälter (z.B. Becomix-Mischer) wird die Hauptmenge des Wassers vorgelegt und die HEC unter Rühren, Homogenisieren und Vakuum eingesaugt und anschließend unter Vakuum für ca. 10 min gerührt. Für ca. 30 min läßt man die HEC bei Raumtemperatur (RT) quellen, bevor man unter Vakuum auf ca. 80 °C erhitzt und für ca. 1 h auf dieser Temperatur hält und wieder auf RT abkühlt.

### 3. Schritt (Natriumbenzoatlösung)

In einem geeigneten Ansatzbehälter (z.B. VA-Behälter) wird ein geringer Teil des Wassers vorgelegt und das Natriumbenzoat unter Rühren darin gelöst.

### 4. Schritt (Endmischung)

Zur Polymerlösung werden die Natriumbenzoat-Lösung (s. o.) und die restlichen Bestandteile der Rezeptur mit Ausnahme des Aromas gegeben (Einsaugen unter Vakuum). Anschließend wird homogenisiert. Danach wird die Wirkstoff/Siliciumdioxid-Mischung unter Vakuum, Rühren und Homogenisieren zugegeben, anschließend wird für weitere 10 min unter Vakuum homogenisiert. Die beschriebenen hohen Scherkräfte sind beispielsweise dadurch charakterisiert, daß das Wirkstoff/Siliciumdioxid-Gemisch bei einem Ansatz von 1000 kg in einem Mischer des Typs Becomix RW 1000 bei einer Rotorgeschwindigkeit von ca. 3500 U/min (entspricht ca. 26 m/s Umfangsgeschwindigkeit) in den Umlauf eingesaugt und dann für 10 min homogenisiert wird. Zuletzt wird unter Vakuum, Rühren und Homogenisieren das Aroma zugesetzt. Diese Methode nutzt die schaumbrechenden Eigenschaften des Aromas, was das anschließende Entlüften der Suspension (Vakuum) verkürzt. Die Suspension kann unter Druck aus dem Mischer in Bulkgefäße abgefüllt werden.

## Patentansprüche

1. Pharmazeutische Zusammensetzung in Form einer oral applizierbaren Suspension umfassend
a) einen Wirkstoff vom NSAID-Typ ausgewählt aus der Gruppe der Propionsäurederivate, der Essigsäurederivate, der Fenaminsäurederivate, der Biphenylcarbonsäurederivate, der sauren Enolcarboxamide, der Diaryl-Heterocyclen mit Methylsulfonyl- oder Aminosulfonyl-Substituenten und der sauren Sulfonamide und deren pharmazeutisch verträglichen Salze, wobei der Wirkstoff ein Komgrößenspektrum aufweist, in dem mindestens 90% der Partikel kleiner als 50 µm sind,
b) ein physiologisch indifferentes Dispersionsmedium, in dem die Löslichkeit des Wirkstoffs geringer als 500 µg/ml ist,
c) 0,1 bis 5 Gew.-% hochdisperses Siliziumdioxid mit einer spezifischen Oberfläche von mindestens 50 m²/g, welches in das Dispersionsmedium unter Anwendung von Scherkräften, charakterisiert durch eine Rotor-Umfangsgeschwindigkeit von 15 bis 35 m/s, eingetragen wird, und
d) 0,05 bis 2 Gew.-% eines wasserlöslichen Celluloseethers in pharmazeutischer Qualität.

2. Pharmazeutische Zusammensetzung gemäß Anspruch 1, **dadurch gekennzeichnet, daß** der Wirkstoff ein saures Enolcarboxamid ausgewählt aus Piroxicam, Tenoxicam, Lornoxicam, Meloxicam und deren pharmazeutisch verträglichen Salzen ist.

3. Pharmazeutische Zusammensetzung gemäß Anspruch 1, **dadurch gekennzeichnet, daß** der wasserlösliche Celluloseether Hydroxyethylcellulose (HEC), Hydroxyproylcellulose (HPC) oder Hydroxypropylmethylcellulose (HPMC) ist.

4. Pharmazeutische Zusammensetzung gemäß einem der Ansprüche 1 oder 3, **dadurch gekennzeichnet, daß** der wasserlösliche Celluloseether in einer Menge von 0,05 bis 0,5 Gew.-% in der Suspension enthalten ist.

5. Pharmazeutische Zusammensetzung gemäß Anspruch 1, **dadurch gekennzeichnet, daß** der Wirkstoff Meloxicam und das physiologisch verträgliche Dispersionsmedium ein wäßriges Puffersystem im pH-Bereich 2-4 ist.

6. Pharmazeutische Zusammensetzung gemäß Anspruch 5, **dadurch gekennzeichnet, daß** das physiologisch verträgliche Dispersionsmedium eine Mischung eines physiologisch verträglichen wäßrigen Puffersystems im pH-Bereich von 2-4 mit Glycerol und / oder wäßrigen Lösungen von Zuckeralkoholen ausgewählt aus Sorbitol, Mannitol und Xylitol und deren Mischungen ist.

7. Pharmazeutische Zusammensetzung nach einem der Ansprüche 5 oder 6, **dadurch gekennzeichnet, daß** das physiologisch verträgliche Dispersionsmedium ein Puffersystem ist, ausgewählt aus:
(a) Natriumdihydrogenphosphat Dihydrat / Citronensäure Monohydrat,
(b) Glycin/HCl,
(c) Na-Citrat/HCl,
(d) K-Hydrogenphthalat/HCl,
(e) Citronensäure/Phosphat,
(f) Citrat-Phosphat-Borat/HCl und
(g) Britton-Robinson Puffer.

8. Pharmazeutische Zusammensetzung gemäß Anspruch 6, **dadurch gekennzeichnet, daß** das Dispersionsmedium aus einer Mischung aus 30 bis 50% wäßrigem Natriumdihydrogenphosphat Dihydrat / Citronensäure Monohydrat-Puffer, 10 bis 20% Glycerol, 10 bis 20% Xylitol und 20 bis 30% Sorbitol- oder Mannitol-Lösung, wobei die Sorbitol- oder Mannitol-Lösung eine 70 %ige wäßrige Lösung ist, besteht.

9. Pharmazeutische Zusammensetzung gemäß Anspruch 1, **dadurch gekennzeichnet, daß** die Suspension als Wirkstoff 0,050 bis 3,000 g/118 g Meloxicam, bezogen auf die Masse der applikationsfertigen Darreichungsform, und gegebenenfalls ein oder mehrere Aromen und/oder ein oder mehrere Süßstoffe und/oder übliche Hilfsstoffe und/oder im pH-Bereich wirksame Konservierungsmittel enthält.

10. Verfahren zur Herstellung einer p.o. applizierbaren, flüssigen Zubereitung eines pharmazeutischen Wirkstoffs des NSAID-Typs in Form einer stabilisierten Suspension, **dadurch gekennzeichnet, daß**
(i) der feste Wirkstoff gemahlen wird, sodaß ein Korngrößenspektrum erzeugt wird, in dem mindestens 90% der Partikel kleiner als 50 um sind,
(ii) der gemahlene Wirkstoff in einem physiologisch indifferenten Dispersionsmedium, in dem die Löslichkeit des Wirkstoffs geringer als 500 µg/ml ist, suspendiert wird,
(iii) in das Dispersionsmedium unter Anwendung hoher Scherkräfte, charakterisiert durch eine Rotor-Umfangsgeschwindigkeit von 15 bis 35 m/s, 0,1 bis 5,0 Gew.-% hochdispersen Siliziumdioxids, bezogen auf das Gewicht der applikationsfertigen Suspension, eingetragen werden,
(iv) dem Dispersionsmedium 0,05 bis 2 Gew.-% eines wasserlöslichen Celluloseethers, bezogen auf das Gewicht der applikationsfertigen Suspension, zugesetzt werden und
(v) dem Dispersionsmedium optional unabhängig voneinander eines oder mehrere Aromen, ein oder mehrere Süßstoffe, übliche Hilfsstoffe oder ein oder mehrere Konservierungsmittel zugesetzt werden.

11. Verfahren gemäß Anspruch 10, **dadurch gekennzeichnet, daß** der Wirkstoff Meloxicam ist und
(i) ein Komgrößenspektrum erzeugt wird, in dem etwa 90% der Partikel kleiner als 10 µm sind,
(ii) der gemahlene Wirkstoff in einem physiologisch verträglichen, wäßrigen Puffersystem im pH-Bereich von 2-4 suspendiert wird,
(iii) mit Hilfe eines Mischers unter Anwendung hoher Scherkräfte, charakterisiert durch eine Rotor-Umfangsgeschwindigkeit von 15 bis 35 m/s, in das Dispersionsmedium 0,1 bis 5,0 Gew.-% hochdisperses Siliziumdioxid, bezogen auf das Gewicht der applikationsfertigen Suspension, eingetragen werden,
(iv) dem Dispersionsmedium als wasserlöslicher Celluloseether Hydroxyethylcellulose in pharmazeutischer Qualität in einer Menge von 0,05 bis 2 Gew.-%, bezogen auf das Gewicht der applikationsfertigen Suspension, zugesetzt wird und
(v) dem Dispersionsmedium optional unabhängig voneinander eines oder mehrere Aromen, ein oder mehrere Süßstoffe, übliche Hilfsstoffe oder ein oder mehrere Konservierungsmittel zugesetzt werden.

## Claims

1. Pharmaceutical composition in the form of a suspension capable of being administered orally, comprising
a) an active substance of the NSAID type selelcted from among the propionic acid derivatives, the acetic acid derivatives, the fenamic acid derivatives, the biphenylcarboxylic acid derivatives, the acid enolcarboxamides, the diaryl heterocycles with methylsulphonyl or aminosulphonyl substituents and the acid sulphonamides and the pharmaceutically acceptable salts thereof, the active substance having a particle size spectrum in which at least 90% of the particles are smaller than 50 µm,
b) a physiologically inert dispersion medium in which the solubility of the active substance is less than 500 µg/ml,
c) 0.1 to 5 wt.% of highly dispersed silicon dioxide with a specific surface area of at least 50 m²/g, which is added to the dispersion medium under the action of shear forces, **characterised by** a circumferential rotor speed of 15 to 35 m/s,
d) 0.05 to 2 wt.% of a pharmaceutical-grade water-soluble cellulose ether.

2. Pharmaceutical composition according to claim 1, **characterised in that** the active substance is an acid enolcarboxamide selected from among piroxicam, tenoxicam, lornoxicam and meloxicam and the pharmaceutically acceptable salts thereof.

3. Pharmaceutical composition according to claim 1, **characterised in that** the water-soluble cellulose ether is hydroxyethyl cellulose (HEC), hydroxypropyl cellulose (HPC) and hydroxypropylmethyl cellulose (HPMC).

4. Pharmaceutical composition according to one of claims 1 or 3, **characterised in that** the water-soluble cellulose ether is present in the suspension in an amount of from 0.05 to 0.5% by weight.

5. Pharmaceutical composition according to claim 1, **characterised in that** the active substance is meloxicam and the physiologically acceptable dispersion medium is an aqueous buffer system with a pH in the range from 2 to 4.

6. Pharmaceutical composition according to claim 5, **characterised in that** the physiologically acceptable dispersion medium is a mixture of a physiologically acceptable aqueous buffer system with a pH in the range from 2 to 4 with glycerol and/or aqueous solutions of sugar alcohols selected from sorbitol, mannitol and xylitol and mixtures thereof.

7. Pharmaceutical composition according to one of claims 5 or 6, **characterised in that** the physiologically acceptable dispersion medium is a buffer system selected from:
(a) sodium dihydrogen phosphate dihydrate/citric acid monohydrate,
(b) glycin/HCl,
(c) Na-citrate/HCl,
(d) K-hydrogen phthalate/HCl,
(e) citric acid/phosphate,
(f) citrate-phosphate-borate/HCl and
(g) Britton-Robinson buffer.

8. Pharmaceutical composition according to claim 6, **characterised in that** the dispersion medium consists of a mixture of 30-50% aqueous sodium dihydrogen phosphate dihydrate/citric acid monohydrate buffer, 10-20% glycerol, 10-20% xylitol and 20-30% sorbitol or mannitol solution, the sorbitol or mannitol solution being a 70% aqueous solution.

9. Pharmaceutical composition according to claim 1, **characterised in that** the suspension contains as active substance 0.050 to 3.000 g/118 g of meloxicam, based on the mass of the preparation ready for use, and optionally one or more flavourings and/or one or more sweeteners and/or conventional excipients and/or preservatives which are effective at the pH range.

10. Process for preparing an orally administered liquid preparation of a pharmaceutical active substance of the NSAID type in the form of a stabilised suspension, **characterised in that**
(i) the solid active substance is ground in order to produce a particle size spectrum in which at least 90% of the particles are smaller than 50 µm,
(ii) the ground active substance is suspended in a physiologically inert dispersion medium in which the solubility of the active substance is less than 500 µg/ml,
(iii) 0.1 to 5.0 wt.% of highly dispersed silicon dioxide, based on the weight of the suspension ready for administration, are added to the dispersion medium with the application of high shear forces, **characterised by** a circumferential rotor speed of 15 to 35 m/s,
(iv) 0.05 to 2 wt.% of a water-soluble cellulose ether, based on the weight of the suspension ready for administration, are added to the dispersion medium and
(v) optionally, one or more flavourings, one or more sweeteners, conventional excipients or one or more preservatives may, independently of one another, be added to the dispersion medium.

11. Process according to claim 10, **characterised in that** the active substance is meloxicam and
(i) a particle size spectrum is produced in which about 90% of the particles are smaller than 10 µm,
(ii) the ground active substance is suspended in a physiologically acceptable, aqueous buffer system at a pH in the range from 2-4,
(iii) with the aid of a mixer by the application of high shear forces, **characterised by** a circumferential rotor speed of 15 to 35 m/s, 0.1-5.0 wt.-% of highly dispersed silicon dioxide, based on the weight of the suspension ready for use, are added to the dispersion medium,
(iv) hydroxyethylcellulose of pharmaceutical grade is added to the dispersion medium as a water-soluble cellulose ether, in an amount of from 0.05 - 2 wt.%, based on the weight of the suspension ready for use, and
(v) one or more flavourings, one or more sweeteners, conventional excipients or one or more preservatives are optionally added independently of one another to the dispersion medium.

## Revendications

1. Composition pharmaceutique sous forme d'une suspension applicable oralement, comprenant
a) un principe actif du type NSAID choisie dans le groupe des dérivés de l'acide propionique, des dérivés de l'acide acétique, des dérivés de l'acide aminofénique, des dérivés de l'acide biphénylcarbonique, des carboxamides énoliques acides, des hétérocyles de diaryle avec des substituants de méthylsulfonyle ou d'aminosulfonyle et des sulfonamides acides et leurs sels pharmaceutiquement compatibles, le principe actif ayant un spectre granulométrique dans lequel au moins 90% des particules sont inférieures à 50 µm,
b) un milieu dispersant physiologiquement indifférent dans lequel la solubilité du principe actif est inférieure à 500 µg/ml,
c) entre 0,1% et 5% en poids de dioxyde de silicium bien dispersé avec une surface spécifique d'au moins 50 m²/g qui est introduit dans le milieu dispersant en appliquant des efforts de cisaillement élevés **caractérisés par** une vitesse périphérique du rotor comprise entre 15 et 35 m/s, et
d) entre 0,05% et 2 % en poids d'un éther de cellulose de qualité pharmaceutique.

2. Composition pharmaceutique selon la revendication 1, **caractérisée en ce que** le principe actif est un acide carboxamide énolique sélectionné parmi le piroxicam, le ténoxicam, le lornoxicam, le méloxicam et leurs sels pharmaceutiquement compatibles.

3. Composition pharmaceutique selon la revendication 1, **caractérisée en ce que** l'éther de cellulose soluble dans l'eau est une cellulose hydroxyéthylique (HEC), une cellulose hydroxyproylique (HPC) ou une cellulose hydroxypropylméthylique (HPMC).

4. Composition pharmaceutique selon l'une des revendications 1 ou 3, **caractérisée en ce que** l'éther de cellulose soluble dans l'eau est contenu dans la suspension en une quantité comprise entre 0,05% et 0,5% en poids.

5. Composition pharmaceutique selon la revendication 1, **caractérisée en ce que** le principe actif méloxicam et le milieu dispersant physiologiquement compatible est un système tampon aqueux dans la plage de pH comprise entre 2 et 4.

6. Composition pharmaceutique selon la revendication 5, **caractérisée en ce que** le milieu dispersant physiologiquement compatible est un mélange d'un système tampon aqueux physiologiquement compatible dans la plage de pH comprise entre 2 et 4 et de glycérol et/ou de solutions aqueuses d'alcools de sucre sélectionnées parmi le sorbitol, le mannitol et le xylitol et leurs mélanges.

7. Composition pharmaceutique selon l'une des revendications 5 ou 6, **caractérisée en ce que** le milieu dispersant physiologiquement compatible est un système tampon sélectionné parmi :
(a) le sodium dihydrogénophosphate dihydraté /l'acide citrique monohydraté,
(b) la glycine/HCl,
(c) le citrate de Na/HCl,
(d) le K-hydrogène phtalate/HCl,
(e) l'acide citrique/phosphate,
(f) le citrate-phosphate-borate/HCl et
(g) le tampon Britton-Robinson.

8. Composition pharmaceutique selon la revendication 6, **caractérisée en ce que** le milieu dispersant est constitué d'un mélange de 30% à 50% de tampon de sodium dihydrogénophosphate dihydraté / d'acide citrique monohydraté, de 10% à 20% de glycérol, de 10% à 20% de xylitol et de 20% à 30% de solution de sorbitol ou de mannitol, la solution de sorbitol ou de mannitol étant une solution aqueuse à 70%.

9. Composition pharmaceutique selon la revendication 1, **caractérisée en ce que** la suspension contient comme principe actif entre 0,050 et 3,000 g/118 g de méloxicam, rapportés à la masse de la forme pharmaceutique prête à l'administration et, éventuellement un ou plusieurs arômes et/ou un ou plusieurs édulcorants et/ou des auxiliaires et/ou des conservateurs actifs dans la plage de pH.

10. Procédé pour la fabrication d'une préparation liquide à administration perorale du type NSAID sous forme d'une suspension stabilisée, **caractérisé en ce que**
(i) le principe actif est moulu afin de produire un spectre granulométrique dans lequel au moins 90% des particules sont inférieures à 50 µm,
(ii) le principe actif moulu est mis en suspension dans un milieu dispersant physiologiquement indifférent dans lequel la solubilité du principe actif est inférieure à 500 µg/ml,
(iii) entre 0,1% et 5,0% en poids de dioxyde de silicium bien dispersé, rapportés au poids de la suspension prête à l'administration, sont introduits dans le milieu dispersant en appliquant des efforts de cisaillement élevés **caractérisés par** une vitesse périphérique du rotor comprise entre 15 et 35 m/s,
(iv) entre 0,05% et 2 % en poids d'un éther de cellulose, rapportés au poids de la suspension prête à l'administration, sont ajoutés au milieu dispersant et
(v) un ou plusieurs arômes, un ou plusieurs édulcorants, des auxiliaires usuels ou un ou plusieurs conservateurs sont ajoutés au milieu dispersant, facultativement indépendamment les uns des autres.

11. Procédé selon la revendication 10, **caractérisé en ce que** le principe actif est le méloxicam et
(i) un spectre granulométrique est obtenu dans lequel environ 90% des particules sont inférieures à 10 µm,
(ii) le principe actif moulu est mis en suspension dans un système tampon aqueux physiologiquement compatible dans une plage de pH comprise entre 2 et 4,
(iii) entre 0,1% et 5,0% en poids de dioxyde de silicium bien dispersé, rapportés au poids de la suspension prête à l'administration, sont introduits dans le milieu dispersant à l'aide d'un mélangeur en utilisant des efforts de cisaillement élevés **caractérisés par** une vitesse périphérique du rotor comprise entre 15 et 35 m/s,
(iv) entre 0,05% et 2 % en poids de cellulose hydroxyéthylique de qualité pharmaceutique, rapportés au poids de la suspension prête à l'administration, sont ajoutés au milieu dispersant comme éther de cellulose soluble dans l'eau et
(v) un ou plusieurs arômes, un ou plusieurs édulcorants, des auxiliaires usuels ou un ou plusieurs conservateurs sont ajoutés au milieu dispersant, facultativement indépendamment les uns des autres.
